# EUROPEAN PATENT APPLICATION

(11) **EP 1 378 502 A1**
(43) Date of publication of application: **07.01.2004**
(21) Application number: 02703972.6
(22) Date of filing: 12.03.2002
(51) Int. Cl.: C07C 69/67, C07C 67/313, C07F 7/18, A61K 31/22

(54) **CHAIN OLIGOLACTIC ACID ESTER**

(30) Priority: 13.03.2001 JP 2001006976
(71) Applicant: AMATO PHARMACEUTICAL PRODUCTS, LTD., Fukuchiyama-shi, Kyoto 620-0932 (JP)
(72) Inventor: WATANABE, Mikio, Hadano-shi, Kanagawa 257-0002 (JP); MURAKAMI, Masahiro, Hirano-ku, Osaka-shi, Osaka 547-0026 (JP)
(74) Representative: Godemeyer, Thomas, Dr.
(86) International application number: PCT/JP2002/002294
(87) International publication number: WO 2002/072531

(57) **Abstract**

An object of the present invention is to produce and isolate an oligolactate ester having a specific chain length as a single compound. The present invention provides a compound represented by formula (1) or a salt thereof. 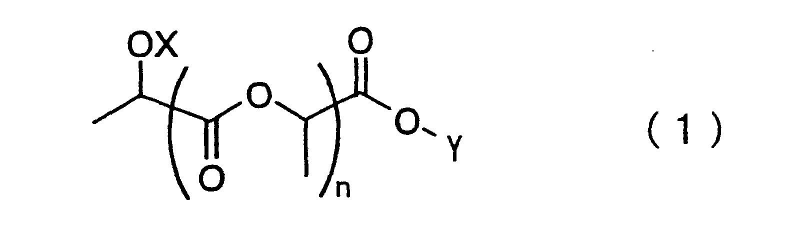 wherein X represents a hydrogen atom or a protecting group of a hydroxyl group, Y represents a lower alkyl group, and n represents an integer of 2 to 19.

## Description

### TECHNICAL FIELD

The present invention relates to a linear oligolactate ester. More particularly, the present invention relates to a purified linear oligolactate ester composed of a single compound, and a method for the preparation of the above mentioned linear oligolactate ester.

### BACKGROUND ART

It has been reported that a cyclic and/or linear poly L-lactate mixture having a condensation degree of 3 to 19 is useful as antineoplastic agent (JP Patent Publications (Kokai) No.9-227388 and No. 10-130153) or a QOL-improving agent for cancer patients (JP Patent Application No. 11-39894; Journal of Japan Society of Clinical Oncology, Vol. 33, 3, pp. 493). Further, it has been revealed that a cyclic and/or linear polylactate mixture having a condensation degree of 3 to 19 has an action of reducing blood sugar level, and thus is useful as a medicament for prevention and/or treatment of diabetes or diabetes complications (JP Patent Application No. 11-224883). Furthermore, it has been revealed that it is useful for controlling excessive appetite, promoting basal metabolism, and improving and/or preventing adiposity.

As described above, it has been demonstrated that a mixture of cyclic and/or linear oligolactate mixture having a condensation degree of 3 to 19 exhibits various pharmaceutical effects, and thus it is expected to be further developed as a medicament in the future. In order to develop an oligolactate as a medicine, it is desirable that a polylactate having a specific chain length is isolated as a single compound rather than as a mixture of polylactates having different chain lengths.

However, it has not been reported so far that a linear oligolactate ester having an esterified terminal carboxyl group and a condensation degree of 3 to 20 has been isolated as a single compound.

### DISCLOSURE OF THE INVENTION

An object to be achieved by the present invention is to produce and isolate an oligolactate ester having a specific chain length as a single compound. Another object to be achieved by the present invention is to provide a method for the preparation of the oligolactate ester having a specific chain length as a single compound.

The present inventors have made intensive efforts to solve the above objects. As a result, they successfully achieved the isolation and identification of linear oligolactate esters from trimers to octamers by reacting an ethyl lactate or a lactoyl ethyl lactate with a lower alkyl-alkali metal, then reacting the resultant with a lactide, and separating the obtained reaction mixture by flash silica gel column chromatography. The present invention has been completed based on this finding.

Namely, the present invention provides a compound represented by formula (1) or a salt thereof. 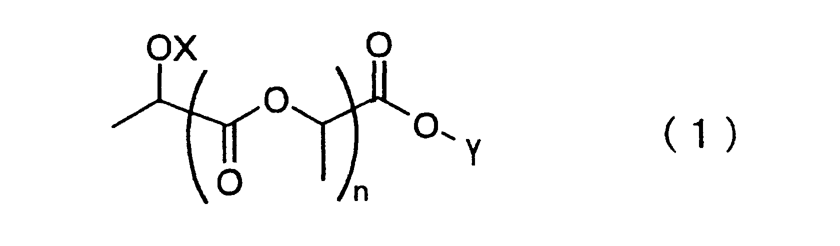 wherein X represents a hydrogen atom or a protecting group of a hydroxyl group, Y represents a lower alkyl group, and n represents an integer of 2 to 19.

Preferably, n represents an integer of 2 to 7.

Preferably, the present invention provides a compound selected from the group consisting of compounds represented by the following formulae (3) to (8) and salts thereof. 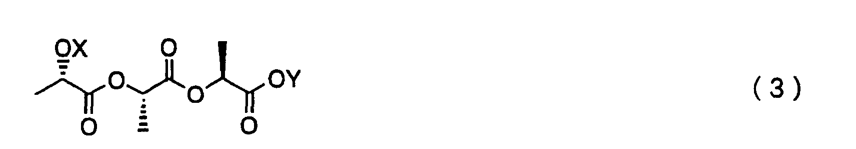 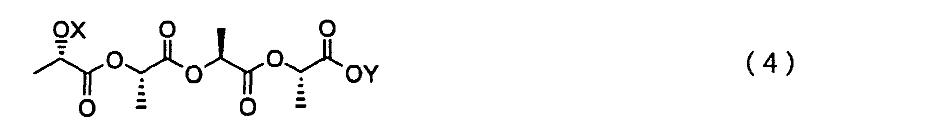 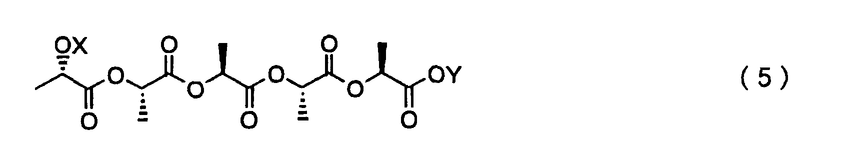 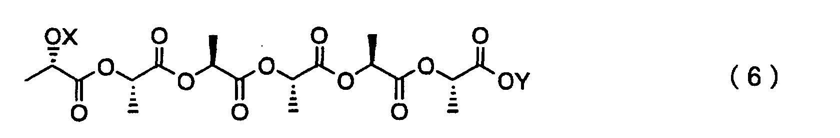 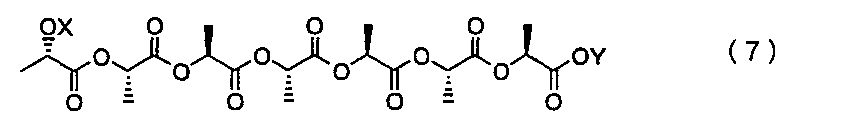 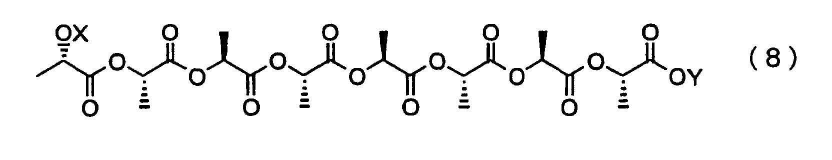 wherein X represents a hydrogen atom or a protecting group of a hydroxyl group and Y represents a lower alkyl group.

According to another aspect of the present invention, there is provided a method for the preparation of the compound represented by the above formula (1) or salts thereof wherein, CH₃CH(OH)COOCH(CH₃)COOY wherein Y represents a lower alkyl group, is allowed to react with a lactide in the presence of a lower alkyl-alkali metal.

According to still another aspect of the present invention, there is provided a method for the preparation of the compound represented by the above formula (1) or salts thereof wherein, CH₃CH(OH)COOY wherein Y represents a lower alkyl group is allowed to react with a lactide in the presence of a lower alkyl-alkali metal.

According to a further another aspect of the present invention, there is provided a method for the preparation of the compound represented by the above formula (1) or salts thereof wherein, CH₃CH(OH)COOCH(CH₃)COOCH(CH₃)COOY wherein Y represents a lower alkyl group, is allowed to react with a lactide in the presence of a lower alkyl-alkali metal.

According to a still further another aspect of the present invention, there is provided a method for the preparation of the compound represented by the above formula (1) or salts thereof wherein a compound represented by the formula (1A) is allowed to react with a lactide in the presence of a lower alkyl-alkali metal: 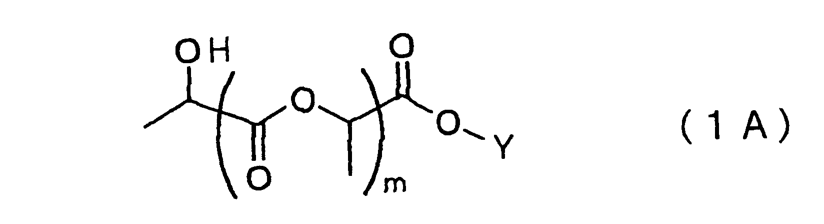 wherein Y represents a lower alkyl group and m represents an integer of 3 to 19.

In the method of the present invention, the product resulting from the reaction with the lactide is separated to obtain a single compound using, preferably chromatography, more preferably column chromatography, and most preferably flash column chromatography.

### BEST MODE FOR CARRYING OUT THE INVENTION

Hereinafter, embodiments of the present invention and methods for carrying out the present invention will be described in detail.

The present invention relates to a compound represented by the formula (1) or salts thereof. 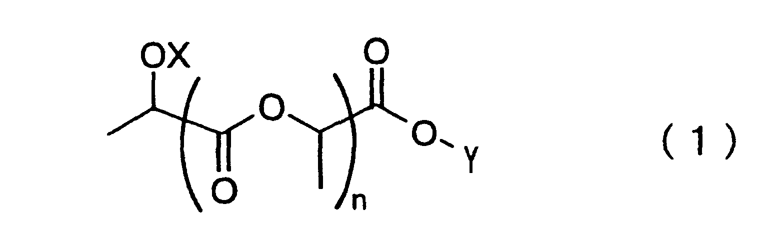 wherein X represents a hydrogen atom or a protecting group of a hydroxyl group, Y represents a lower alkyl group, and n represents an integer of 2 to 19.

The type of a protecting group of the hydroxyl group represented by X is not particularly limited, and a person skilled in the art can appropriately select the type thereof. Examples of the protecting group of the hydroxyl group are listed below.

### (ether type)

methyl group, methoxymethyl group, methylthiomethyl group, benzyloxymethyl group, t-butoxymethyl group, 2-methoxyethoxymethyl group, 2,2,2-trichloroethoxymethyl group, bis(2-chloroethoxy)methyl group, 2-(trimethylsilyl)ethoxymethyl group, tetrahydropyranyl group, 3-bromotetrahydropyranyl group, tetrahydrothiopyranyl group, 4-methoxytetrahydropyranyl group, 4-methoxytetrahydrothiopyranyl group, 4-methoxytetrahydrothiopyranyl S,S-dioxide group, tetrahydrofuranyl group, and tetrahydrothiofuranyl group;
1-ethoxyethyl group, 1-methyl-1-methoxyethyl group, 1-(isopropoxy)ethyl group, 2,2,2-trichloroethyl group, 2-(phenylselenyl)ethyl group, t-butyl group, allyl group, cinnamyl group, p-chlorophenyl group, benzyl group, p-methoxybenzyl group, o-nitrobenzyl group, p-nitrobenzyl group, p-halobenzyl group, p-cyanobenzyl group, 3-methyl-2-picolyl-N-oxido group, diphenylmethyl group, 5-dibenzosuberyl group, triphenylmethyl group, α -naphthyldiphenylmethyl group, p-methoxyphenyldiphenylmethyl group, p-(p'-bromophenacyloxy)phenyldiphenylmethyl group, 9-anthryl group, 9-(9-phenyl)xanthenyl group, 9-(9-phenyl-10-oxo)anthryl group, and benzisothiazolyl S,S-dioxido group;
trimethylsilyl group, triethylsilyl group, isopropyldimethylsilyl group, t-butyldimethylsilyl group (TBDMS group), (triphenylmethyl)dimethylsilyl group, t-butyldiphenylsilyl group, methyldiisopropylsilyl group, methyldi-t-butylsilyl group, tribenzylsilyl group, tri-p-xylylsilyl group, triisopropylsilyl group, and triphenylsilyl group;

### (ester type)

formate, benzoylformate, acetate, chloroacetate, dichloroacetate, trichloroacetate, trifluoroacetate, methoxyacetate, triphenylmethoxyacetate, phenoxyacetate, p-chlorophenoxyacetate, 2,6-dichloro-4-methylphenoxyacetate, 2,6-dichloro-4-( 1,1,3,3-tetramethylbutyl)phenoxyacetate,
2,4-bis(1,1-dimethylpropyl)phenoxyacetate, chlorodiphenylacetate, p-P-phenylacetate, 3-phenylpropionate, 3-bezoylpropionate, isobutyrate, monosccinoate, 4-oxopentanoate, pivaloate, adamantoate, crotonate, 4-methoxycrotonate, (E)-2-methyl-2-butenoate, benzoate, o-(dibromomethyl)benzoate, o-(methoxycarbonyl)benzoate, p-phenylbenzoate, 2,4,6-trimethylbenzoate, p-P-benzoate, and α -naphtoate

### (carbonate type)

methyl carbonate, ethyl carbonate, 2,2,2-trichloroethyl carbonate, isobutyl carbonate, vinyl carbonate, allyl carbonate; cinnamyl carbonate, p-nitrophenyl carbonate, benzyl carbonate, p-methoxybenzyl carbonate, 3,4-dimethoxybenzyl carbonate, o-nitrobenzyl carbonate, p-nitrobenzyl carbonate, and S-benzyl thiocarbonate (others)

N-phenylcarbamate, N-imidazolylcarbamate, borate, nitrate, N,N,N',N'-tetramethylphosphorodiamidate, and 2,4-dinitrophenyl-sulfenate

A method for introducing a protecting group as described above and a method for deprotection are known to those skilled in the art, and are described in, for example, Teodora, W.Green, Protective Groups in Organic Synthesis, John & Wiley & Sons Inc. (1981), and the like.

In the present specification, the carbon number of a lower alkyl group represented by Y is not particularly limited, but the number is generally 1 to 10, preferably 1 to 6, and more preferably 1 to 4. In addition, the chain type of the lower alkyl group is not particularly limited, and any of a straight chain, a branched chain, a cyclic chain or a combination thereof is usable. Examples of the lower alkyl group include methyl group, ethyl group, propyl group, isopropyl group, cyclopropyl group, butyl group, isobutyl group, sec-butyl group, tert-butyl group, cyclopropylmethyl group, cyclobutyl group, pentyl group, and hexyl group.

"n" represents an integer of 2 to 19, preferably 2 to 7. When n is 2, lactic acid is obtained as a trimer. When n is 3, lactic acid is obtained as a tetramer. In the same manner, when n is 19, lactic acid is obtained as an icosamer.

The compound of the present invention is not obtained as a mixture of oligolactate esters having different chain lengths (that is, compounds wherein n in the formula (1) has different numbers), but is obtained as a single substance of oligolactate esters having a specific chain length (that is, compounds wherein n has a constant value).

When X is a hydrogen atom in the formula (1), the compound of the present invention can exist also as a metal salt. Examples of such metal salt include alkali metal salts such as sodium salts and potassium salts, alkaline-earth metal salts such as magnesium salts and calcium salts, aluminum salts, and zinc salts.

Further, various hydrates, solvates or crystal polymorph substances of the compound of the present invention are also within the scope of the invention.

Since the compound of the present invention contains asymmetric carbon(s), the compound has stereoisomers, and all possible isomers and mixtures containing two or more kinds of the isomers at an arbitrary ratio are also within the scope of the invention. Namely, the compound of the present invention includes mixtures of various optical isomers such as optically active substances, racemates, and diastereomers, and isolates thereof.

The configuration of the compound of the present invention is dependent on the configuration of a lactic acid unit in a compound used as a starting material. That is, depending on which is selected among L form, D form or mixtures thereof as the lactic acid unit in the compound used as a starting material, the configuration of the compound of the present invention is diversified. In the present invention, it is preferable to use L form as the configuration of the lactic acid unit.

For example, when L-lactide is used as a starting material, the following compound is obtained.

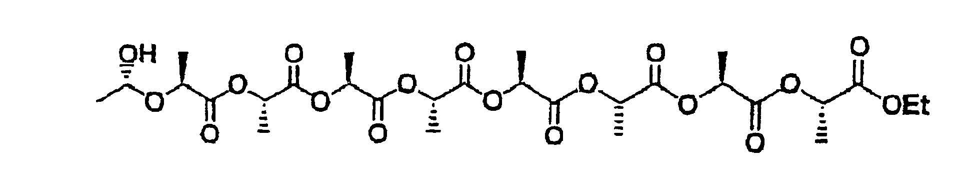

Further, when D-lactide is used as a starting material, the following compound is obtained.

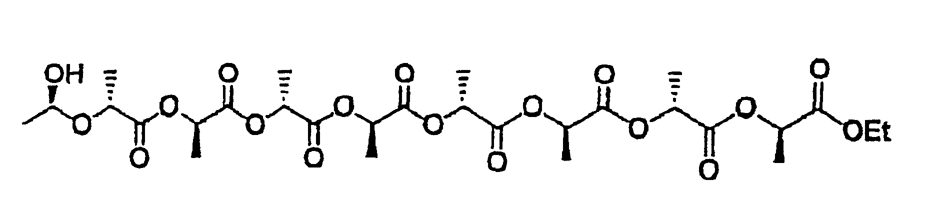

Furthermore, when D, L-lactide is used as a starting material, the following compound is obtained.

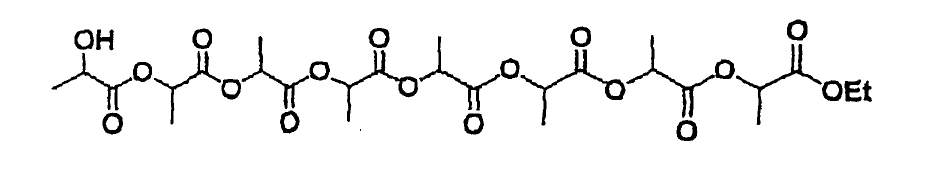

Next, a method for the preparation of the compound of the present invention or salts thereof will be described.

The compound of the present invention can be produced by reacting ester compounds represented by CH₃CH(OH)COOCH(CH₃)COOY, CH₃CH(OH)COOY, or CH₃CH(OH)COOCH(CH₃)COOCH(CH₃)COOY (wherein Y represents a lower alkyl group) with a lactide in the presence of a lower alkyl-alkali metal. The reaction product obtained by the above reaction is usually a mixture of lactic acid oligomers having different chain lengths, and therefore the separation of the product using flash column chromatography allows the isolation and purification of a single compound of the present invention.

A method for obtaining CH₃CH(OH)COOCH(CH₃)COOY (wherein Y represents a lower alkyl group), which is used as a starting material, is not particularly limited, but it can be produced, for example, by causing the ring opening of a lactide in the presence of a metal alkoxide.

Examples of the metal alkoxide which is used herein include those of a lower alcohol (methanol, ethanol, propanol, butanol, pentanol, hexanol, etc.) and an alkali metal (e.g., Li, Na, or K). A method for obtaining the metal alkoxide is not particularly limited, and it can be appropriately obtained by a person skilled in the art. It can be obtained, for example, by reacting a lower alcohol (e.g., ethanol etc.) with an alkyl-alkali metal (e.g., n-butyllithium etc.).

CH₃CH(OH)COOY (wherein Y represents a lower alkyl group) which is used as a starting material is a publicly known compound, and it can be easily synthesized from lactic acid and a lower alcohol.

CH₃CH(OH)COOCH(CH₃)COOCH(CH₃)COOY which is used as a starting material is a compound which falls under the scope of the present invention. It can be obtained by using CH₃CH(OH)COOCH(CH₃)COOY or CH₃CH(OH)COOY as a starting material as described above, and reacting it with a lactide in the presence of a lower alkyl-alkali metal.

The lower alkyl-alkali metal used in the method of the present invention is defined as a compound represented by the following formula: R-Me. In the formula, R represents a lower alkyl group and Me represents an alkali metal. The carbon number of the lower alkyl group represented by R is not limited, but the number is generally 1 to 10, preferably 1 to 6, and more preferably 1 to 4. Further, the chain type of the lower alkyl group is not limited, and any of a straight chain, a branched chain, a cyclic chain, or a combination thereof is usable. Examples of the lower alkyl group include methyl group, ethyl group, propyl group, isopropyl group, cyclopropyl group, butyl group, isobutyl group, sec-butyl group, tert-butyl group, cyclopropylmethyl group, cyclobutyl group, pentyl group, and hexyl group. Examples of the alkali metals represented by Me include Li, Na, and K, and Li is preferred.

When an alkali hydroxy ester is reacted with a lactide in the presence of a lower alkyl alkali metal according to the method of the present invention, the amount of each compound to be used is not particularly limited and can be appropriately determined. The ratio between the alkali hydroxy ester and the lower alkyl alkali metal is preferably 1:0.1-1:10, and more preferably 1:0.5-1:2. In addition, the ratio between the alkali hydroxy ester and the lactide is preferably 1:0.1-1:10, and more preferably 1:0.5-1:2.

The reaction temperature for the method of the present invention is not particularly limited as long as the reaction proceeds. However, the temperature is preferably from -100°C to room temperature, and more preferably -78°C to 0°C.

The reaction according to the present invention is conducted preferably in the presence of a reaction solvent. The reaction solvent is not particularly limited as long as it is inactive to the reaction. Preferably, cyclic ethers such as tetrahydrofran (THF), diethyl ether, dimethoxyethane or the like can be used.

Further, an inactive gas atmosphere such as nitrogen gas or argon gas can be used as a reaction atmosphere.

Hereinafter, one preferable embodiment of the present invention will be described in detail.

Under an inactive atmosphere such as a nitrogen gas atmosphere, at a temperature of -100°C to room temperature, preferably -78°C to -50°C, n-BuLi is added to a THF solution containing an ester compound represented by CH₃CH(OH)COOCH(CH₃)COOY, CH₃CH(OH)COOY or CH₃CH(OH)COOCH(CH₃)COOCH(CH₃)COOY (wherein Y represents a lower alkyl group), and the mixture is stirred for a certain period. Further, a THF solution containing L-(-)-lactide is added thereto and the mixture is stirred for a certain period. To the reaction mixture, saturated ammonium chloride aqueous solution is added, and ether extraction is conducted. Thereafter, the extracted product is washed with saturated saline and dried using sodium sulfate. After filtration, the solvent is removed by an evaporator, so that oily matter can be obtained.

Next, under an inactive atmosphere such as nitrogen gas atmosphere at room temperature, a methylene chloride solution containing t-butyldimethylsilane chloride (TBDMS-Cl) is added to a methylene chloride solution containing imidazol, and the mixture is stirred for a certain period. Further, a methylene chloride solution containing the oily matter obtained above is added thereto, and the mixture is stirred for a certain period. Water and ether are added to the reaction mixture for liquid separation, and an organic layer is washed with saturated saline and then dried with sodium sulfate. After filtration, the solvent is removed by an evaporator.

The reaction product obtained by the above reaction is usually a mixture of lactic acid oligomers having different chain lengths, and therefore it is separated by flash column chromatography according to the production method of the present invention. The flash column chromatography is preferably a flash silica gel column chromatography.

A developing solvent may be, for example, ether : hexane = 1:10 to 1:2. Trimers or icosamers of ethyl ester can be obtained from an eluate thereof.

According to the present invention, any single ethyl ester selected from ethyl ester trimers to icosamers obtained by the above method can be further used for the next reaction as a starting material. Namely, this single ester compound is reacted with a lactide in the presence of a lower alkyl alkali metal, and thereby a linear oligolactate ester having a much larger molecular weight (that is, higher condensation) can be synthesized and isolated.

All of the contents disclosed in the specification of Japanese Patent Application No. 2001-69766, based on which the present application claims a priority, are incorporated herein as a part of the disclosure of this specification.

The present invention will be described in detail by the following examples, but the present invention is not limited thereto.

### EXAMPLES

### Example 1: Synthesis of TBDMS (1) of lactoyl ethyl lactate

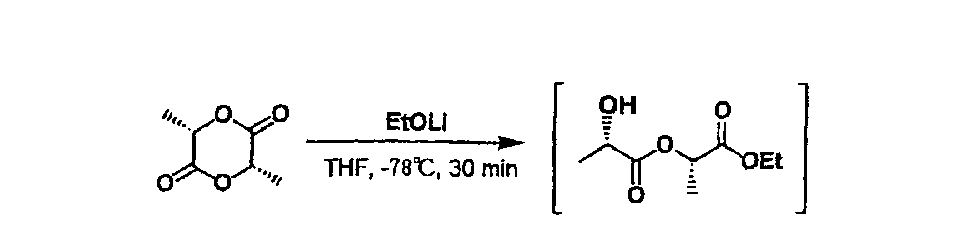

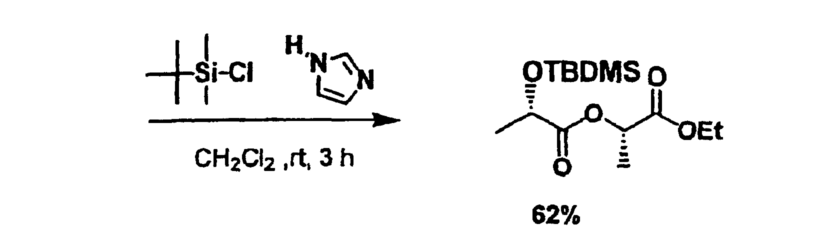

Under a nitrogen gas atmosphere, 3.75 ml (6 mmol) of n-BuLi (1.6 M hexane solution) was added to 15 ml of THF solution containing 0.280 g (6 mmol) of ethanol at -78°C, and the mixture was stirred for 5 minutes to obtain lithium ethoxide. To the obtained solution, 12 ml of THF containing 0.862 g (6 mmol) of L-(-)-lactide was added, and the mixture was stirred and reacted for 30 minutes. The reaction was terminated by adding saturated ammonium chloride aqueous solution thereto, and ether extractions (30 ml x 3 times) were conducted. An ether layer was washed with 20 ml of saturated saline, and thereafter dried with sodium sulfate for 1.5 hours. Sodium sulfate was removed by filtration, and an organic solvent was removed by an evaporator, thereby obtaining oily matter. The obtained oily matter I was directly used for the next reaction.

Under a nitrogen gas atmosphere, 1.085 g of t-butyldimethylsilane chloride (TBDMS-Cl) was added to 20 ml of methylene chloride solution containing 1.021 g (15 mmol) of imidazol, and the mixture was stirred for 15 minutes. To the reaction solution, 15 ml of methylene chloride solution containing the oily matter I (0.9858 g) obtained above was dropwisely added and the mixture was stirred and reacted for 15 hours. The reaction mixture was transferred to a separatory funnel, and 50 ml of water and 100 ml of ether were added thereto for liquid separation. An ether layer was washed with saturated saline, and thereafter was dried with sodium sulfate for 1.5 hours. After sodium sulfate was separated by filtration, an organic solvent was removed by an evaporator, thereby obtaining oily matter II. This obtained oily matter II were separated by flash silica gel column chromatography. Using ether : hexane (1:10) as a developing solvent, O-t-butyldimethylsiloxy lactoyl ethyl lactate (1) was obtained with a yield of 62%.
¹H-NMR (300MHz, CDCl₂), d ppm=0.07 (3H, s), 0.09 (3H, s), 0.885 (9H, s), 1.244 (3H, t, J=6.9 Hz), 1.43 (3H, d, J=6.9 Hz), 1.48 (3H, d, J=6.9 Hz), 4.166 (2H, q, J=6.9 Hz), 4.377 (1H, q, J=6.9 Hz), 5.072 (1H, q, J=6.9 Hz)

### Example 2: Synthesis (deprotection) of lactoyl ethyl lactate (2)

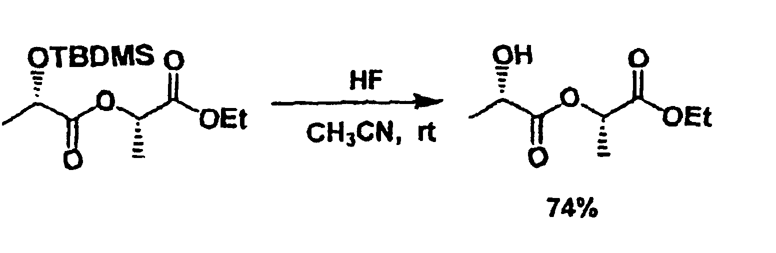

At room temperature, 9 drops of hydrofluoric acid (46%) were added to 12 ml of acetonitrile solution containing 0.9858 g of O-t-butyldimethylsiloxy lactoyl ethyl lactate (1) in a 30 ml double-cap eggplant-flask, and the mixture was stirred for 1 hour. Saturated sodium bicarbonate solution was added to the resultant mixture for the treatment of the reaction, and then extractions were conducted with 30 ml of ether three times. Thereafter, the obtained mixture was washed with saturated saline and dried with sodium sulfate for 1.5 hours. After filtration, the solvent was removed by an evaporator. Pure lactoyl ethyl lactate (2) was obtained with a yield of 76% using flash silica gel column chromatography (developing solvent (ether:hexane=2:1)).
¹H-NMR (300MHz, CDCl₂) d ppm=1.26 (3H, t, J=6.9 Hz), 1.48 (3H, d, J=6.9 Hz), 1.51 (3H, d, J=6.9 Hz), 4.19 (2H, q, J=6.9 Hz), 4.34 (1H, q, J=6.9 Hz), 5.15 (1H, q, J=6.9 Hz)

### Example 3: Reaction between lactoyl ethyl lactate (1) and lactide

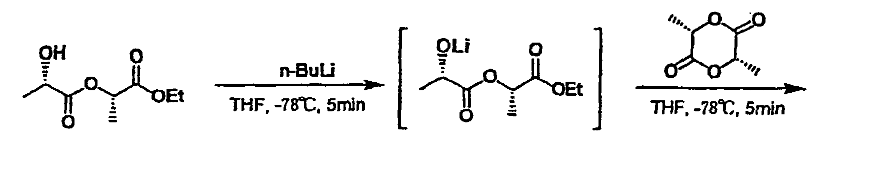

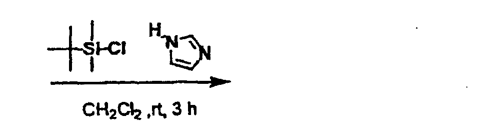

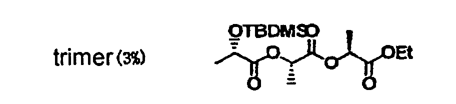

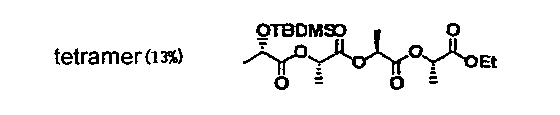

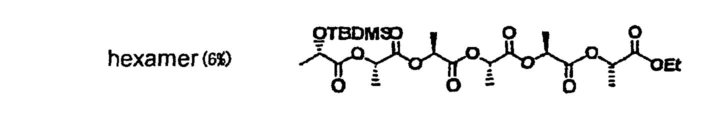

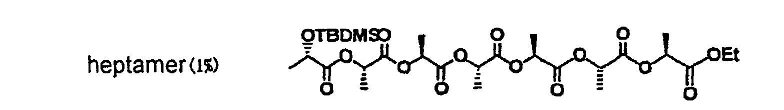

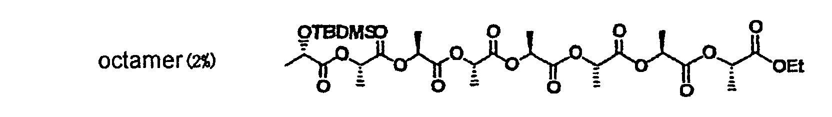

Under a nitrogen gas atmosphere, 3.75 ml (6 mmol) of n-BuLi was added to 15 ml of THF containing 1.1462 g (6 mmol) of lactoyl ethyl lactate at -78°C, and the mixture was stirred for 5 minutes. Further, 12 ml of THF containing 0.862 g (6 mmol) of L-(-)-lactide was added thereto, and the mixture was stirred for 5 minutes. Saturated ammonium chloride aqueous solution was added thereto, and ether extractions were conducted three times. Thereafter, the extracted product was washed with saturated saline, and dried with sodium sulfate for 2 hours. After filtration, the solvent was removed by an evaporator, thereby obtaining oily matter III.

Under a nitrogen gas atmosphere, 20 ml of methylene chloride solution containing 1.085 g of TBDMS-Cl was added to 20 ml of methylene chloride containing 1.022 g (15 mmol) of imidazol at room temperature, and the mixture was stirred for 15 minutes. Further, 15 ml of methylene chloride containing 1.8334 g of the oily matter III was added thereto, and the mixture was stirred for 15 hours. Then, 50 ml of water and 150 ml of ether were added thereto for liquid separation. An organic layer was washed with saturated saline water, and dried with sodium sulfate for 1.5 hours. After filtration, the solvent was removed by an evaporator.

The obtained product was separated using flash silica gel column chromatography with a developing solvent (ether:hexane=1:10-1:2), and trimers of ethyl ester TBDMS and tetramers of ethyl ester TBDMS were obtained from an eluate with yields of 3% and 13%, respectively. Hexamers of ethyl ester TBDMS, heptamers of ethyl ester TBDMS, and octamers of ethyl ester TBDMS were obtained with yields of 6%, 1%, and 2%, respectively.

### Tetramers of ethyl ester TBDMS

¹H-NMR (300MHz, CDCl₂), d ppm=0.07 (3H, s), 0.10 (3H, s), 0.89 (9H, s), 1.26 (3H, t, J=6.9 Hz), 1.44 (3H, d, J=6.9 Hz), 1.50 (3H, d, J=6.9 Hz), 1.57 (6H, dd, J=10.5 Hz), 4.18 (2H, q, J=6.9 Hz), 4.39 (1H, q, J=6.9 Hz), 5.07-5.21(3H, m)

### Trimers of ethyl ester TBDMS

¹H-NMR (300 MHz, CDCl₂), d ppm=0.08 (3H, s), 0.10 (3H, s), 0.90 (9H, s), 1.26 (3H, t, J=6.9 Hz), 1.44 (3H, d, J=6.6 Hz), 1.50 (3H, d, J=6.9 Hz), 1.58 (3H, d, J=6.9 Hz), 4.18 (2H, q, J=6.9 Hz), 4.40 (1H, q, J=6.9 Hz), 5.13 (2H, q, J=6.9 Hz)

### Hexamers of ethyl ester TBDMS

¹H-NMR (300 MHz, CDCl₂), d ppm=0.08 (3H, s), 0.10 (3H, s), 0.90 (9H, s), 1.26 (3H, t, J=6.9 Hz), 1.44 (2H, d, J=6.9 Hz), 1.51 1 (2H, d, J=6.9 Hz), 1.56-1.60 (4H, m), 4.18 (2H, q, J=6.9 Hz), 4.39 (1H, q, J=6.9 Hz), 5.08-5.20 (5H, m)

### Heptamers of ethyl ester TBDMS

¹H-NMR (300 MHz, CDCl₂), d ppm=0.08 (3H, s), 0.10 (3H, s), 0.89 (9H, s), 1.26 (3H, t, J=13.8 Hz), 1.44 (3H, d, J=6.6 Hz), 1.51 (3H, d, J=6.9 Hz), 1.56-1.59 (5H, m), 4.19 (2H, q, J=21.3 Hz), 4.39 (1H, q, J=20.1 Hz), 5.09-5.21 (6H, m)

### Octamers of ethyl ester TBDMS

¹H-NMR (300 MHz, CDCl₂), d ppm=0.07 (3H, s), 0.09 (3H, s), 0.89 (9H, s), 1.25 (3H, t, J=6.9 Hz), 1.43 (2H, d, J=6.9 Hz), 1.49 (2H, d, J=6.9 Hz), 1.56-1.58 (6H, m), 4.18 (2H, q, J=6.9 Hz), 4.39 (1H, q, J=6.9 Hz), 5.08-5.20 (7H, m)

### Example 4: Reaction of lactide and lithium salt of ethyl lactate

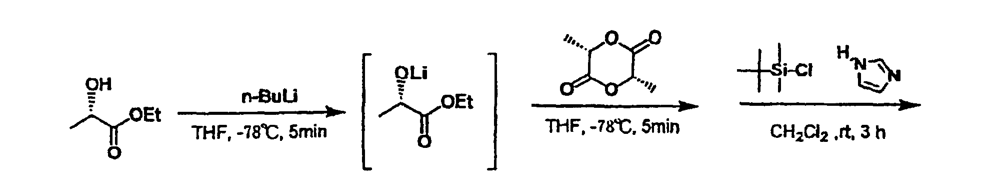

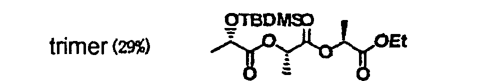

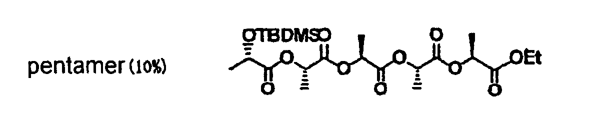

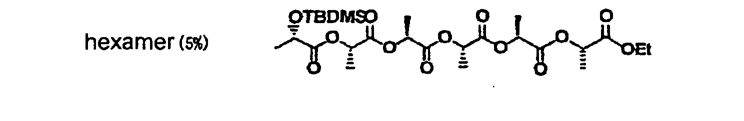

Under a nitrogen gas atmosphere, 2.5 ml (4 mmol) of n-BuLi (1.6M hexane solution) was added to 25 ml of THF containing 0.472 g (4 mmol) of ethyl lactate at -78°C, and the mixture was stirred for 5 minutes. Further, 10 ml of THF containing 0.577 g (4 mmol) of L-(-)-lactide was added thereto, and the mixture was stirred for 5 minutes. Saturated ammonium chloride aqueous solution was added thereto, and ether extractions were conducted three times. Thereafter, the extracted product was washed with saturated saline, and dried with sodium sulfate for 2 hours. After filtration, the solvent was removed by an evaporator, thereby obtaining oily matter IV.

Under a nitrogen gas atmosphere, 20 ml of methylene chloride containing 0.724 g (4.8 mmol) of TBDMS-Cl was added to 24 ml of methylene chloride containing 0.680 g (10 mmol) of imidazol at room temperature, and the mixture was stirred for 30 minutes. Further, 10 ml of methylene chloride containing the oily matter IV was dropwisely added thereto, and the mixture was stirred for 3 hours. Thereafter, 50 ml of water and 150 ml of ether were added thereto for liquid separation. An organic layer was washed with saturated saline water, and dried with sodium sulfate for 1.5 hours. After filtration, the solvent was removed by an evaporator.

The obtained product was separated using flash silica gel column chromatography with a developing solvent (ether:hexane=1:8), and trimers of ethyl ester TBDMS was obtained from an eluate with a yield of 29%. As other type of TBDMSs, pentamers and hexamers were obtained with yields of 10% and 5%, respectively.

### Synthesis (deprotection) of trimers of ethyl ester (4)

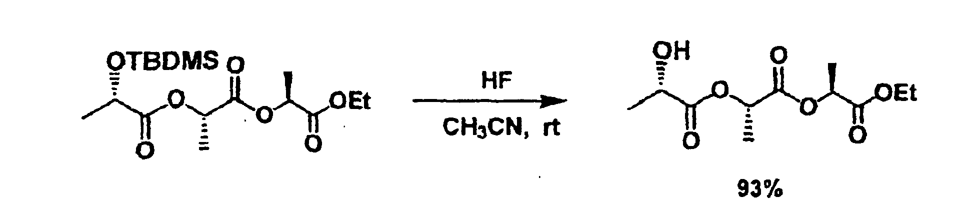

At room temperature, 4 drops of hydrofluoric acid (46%) were added to 5 ml of acetonitril containing trimers of ethyl ester TBDMS in a 30-ml eggplant-shaped flask, and the mixture was stirred for 3 hours. Saturated sodium bicarbonate solution was added thereto, and ether extractions were conducted three times. Thereafter, the extracted product was washed with saturated saline and dried with sodium sulfate for 2 hours. After filtration, the solvent was removed by an evaporator.

The obtained product was purified using flash column chromatography (developing solvent (ether:hexane=2:1)), and then trimers of ethyl ester were obtained with a yield of 93%.

### Example 5: Reaction between lithium salt of trimer of ethyl ester and lactide

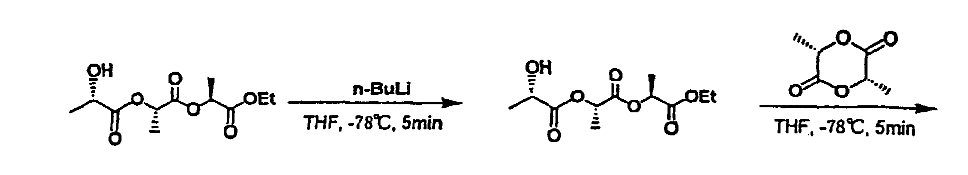

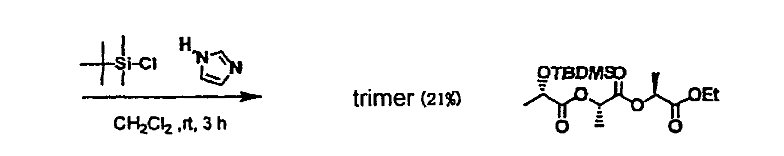

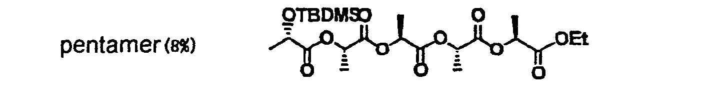

Under a nitrogen gas atmosphere, 0.65 ml (1 mmol) of n-BuLi (1.6 M hexane solution) was added to 25 ml of THF containing 0.274 g (1 mmol) of lactic acid ethyl ester at -78°C, and the mixture was stirred for 5 minutes. Then, 10 ml of THF containing 0.151 g (1 mmol) of L-(-)-lactide was added thereto, and the mixture was stirred for 5 minutes. Saturated ammonium chloride aqueous solution was added thereto, and ether extractions were conducted three times. Thereafter, the extracted product was washed with saturated saline and dried with sodium sulfate for 2 hours. After filtration, the solvent was removed by an evaporator, thereby obtaining oily matter V.

Under a nitrogen gas atmosphere, 20 ml of methylene chloride containing 0.210 g of TBDMS-Cl was added to 24 ml of methylene chloride containing 0.213 g (2.5 mmol) of imidazol at room temperature, and the mixture was stirred for 30 minutes. Further, 10 ml of methylene chloride containing the oily matter V was added thereto, and the mixture was stirred for 3 hours. Thereafter, 50 ml of water and 150 ml of ether were added for liquid separation. An organic layer was washed with saturated saline water and dried with sodium sulfated for 1.5 hours. After filtration, the solvent was removed by an evaporator.

The obtained product was separated using flash column chromatography with a developing solvent (ether:hexane=1:8), and pentamers of ethyl ester TBDMS were obtained from an eluate with a yield of 21%. As other type of TBDMSs, dimmers, trimers (starting material), and hexamers were obtained with yields of 13%, 41 %, and 8%, respectively.

### Pentamers of ethyl ester TBDMS

¹H-NMR (300MHz, CDCl₂), d ppm=0.08 (3H, s), 0.11 (3H, s), 0.90 (9H, s), 1.27 (3H, t, J=6.9 Hz), 1.44 (3H, d, J=6.9 Hz), 1.51 (3H, d, J=6.9 Hz), 1.56-1.59 (3H, m), 4.19 (2H, q, J=6.9 Hz), 4.39 (1H, q, J=6.9 Hz), 5.08-5.21 (3H, m)

### INDUSTRIAL APPLICABILITY

According to the present invention, an oligolactate ester having a specific chain length can be provided as a single compound. Using the single compound of oligolactate ester provided by the present invention, it is possible to develop the oligolactate ester as a medicine, a starting material for medicine, a food additive, a starting material for scented cosmetics, a starting material for formulation, and an additive for formulation.

## Claims

1. A compound represented by formula (1) or a salt thereof. 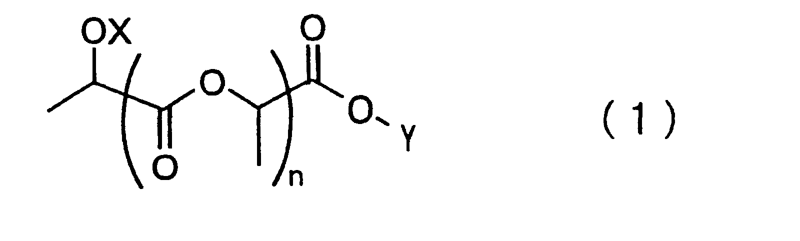 wherein X represents a hydrogen atom or a protecting group of a hydroxyl group, Y represents a lower alkyl group, and n represents an integer of 2 to 19.

2. The compound according to claim 1 or a salt thereof wherein n represents an integer of 2 to 7.

3. A compound selected from the group consisting of compounds represented by the following formulae (3) to (8) and salts thereof. 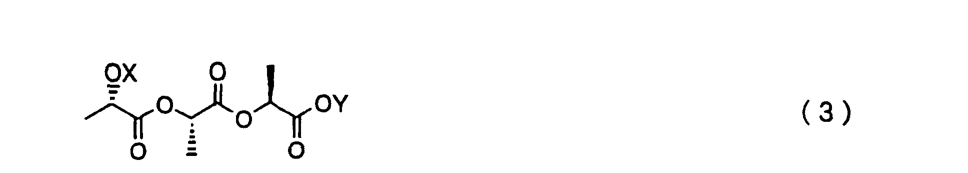 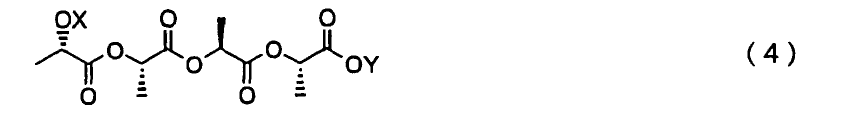 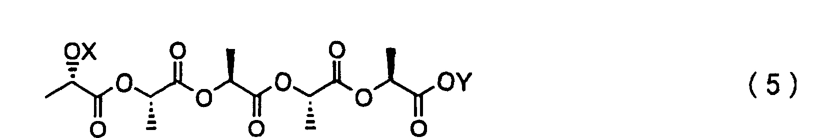 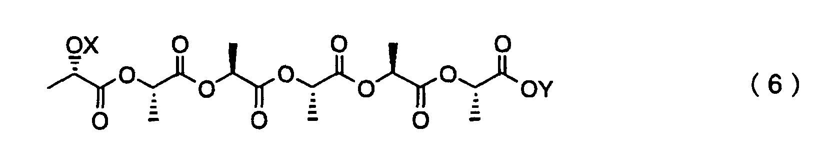 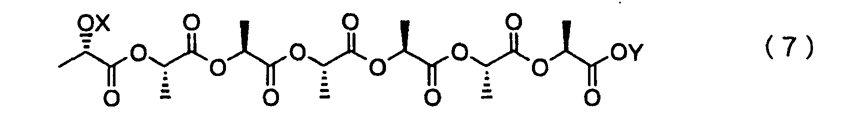 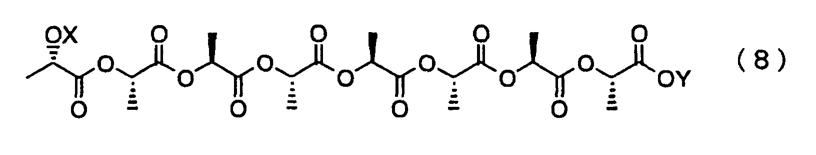 wherein X represents a hydrogen atom or a protecting group of a hydroxyl group and Y represents a lower alkyl group.

4. A method for the preparation of the compound according to any of claims 1 to 3 or salts thereof wherein, CH₃CH(OH)COOCH(CH₃)COOY wherein Y represents a lower alkyl group, is allowed to react with a lactide in the presence of a lower alkyl-alkali metal.

5. A method for the preparation of the compound according to any of claims 1 to 3 or salts thereof wherein, CH₃CH(OH)COOY wherein Y represents a lower alkyl group is allowed to react with a lactide in the presence of a lower alkyl-alkali metal.

6. A method for the preparation of the compound according to any of claims 1 to 3 or salts thereof wherein, CH₃CH(OH)COOCH(CH₃)COOCH(CH₃)COOY wherein Y represents a lower alkyl group, is allowed to react with a lactide in the presence of a lower alkyl-alkali metal.

7. A method for the preparation of the compound according to any of claims 1 to 3 or salts thereof wherein a compound represented by the formula (1A) is allowed to react with a lactide in the presence of a lower alkyl-alkali metal: 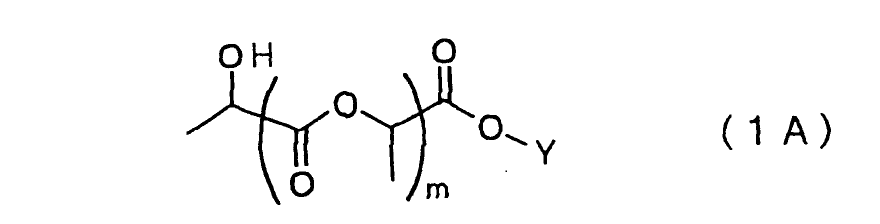 wherein Y represents a lower alkyl group and m represents an integer of 3 to 19.

8. The method according to any of claims 4 to 7, wherein the product resulting from the reaction with the lactide is separated to obtain a single compound using chromatography.

9. The method according to claim 8 wherein chromatography is carried out by column chromatography.

10. The method according to claim 8 wherein chromatography is carried out by flash column chromatography.
